# FASCICULE DE BREVET EUROPEEN

(11) **EP 0 567 379 B1**
(45) Date de publication et mention de la délivrance du brevet: **23.07.1997**
(21) Numéro de dépôt: 93400998.6
(22) Date de dépôt: 16.04.1993
(51) Int. Cl.: G02F 1/35, C08F 8/14, C08F 265/04, C08F 120/18, C07D 307/68

(54) **Matériau réticulé photochimiquement pour application à l'optique non linéaire**
Fotochemisch vernetztes Material für nichtlineare optische Anwendung
Photochemically crosslinked material for non-linear optical application

(30) Priorité: 22.04.1992 FR 9204925
(43) Date de publication de la demande: 27.10.1993
(73) Titulaire: THOMSON-CSF, 75008 Paris (FR)
(72) Inventeur: Muller, Sophie, F-92402 Courbevoie Cédex (FR); Le Barny, Pierre, F-92402 Courbevoie Cédex (FR); Chastaing, Evelyne, F-92402 Courbevoie Cédex (FR); Dubois, Jean-Claude, F-92402 Courbevoie Cédex (FR)
(74) Mandataire: Guérin, Michel

(56) Documents cités:
- EP-A- 0 220 042
- EP-A- 0 482 985
- SOVIET JOURNAL OF QUANTUM ELECTRONICS, AMERICAN INSTITUTE OF PHYSICS, NEW-YORK, 1977 vol. 7, no. 1, Janvier 1977, pages 129 - 131 B. L. DAVIDOFF ET AL. ''New nonlinear organic materials for generation of the second harmonic of neodymium laser radiation''

## Description

La présente invention concerne des matériaux polymères photoréticulables utilisables en optique non linéaire et notamment dans la réalisation de composants pour l'optique intégrée tels qu'un doubleur de fréquence opérant à des longueurs d'onde comprises entre 0,8 et 2µm ou un modulateur électrooptique utilisant une onde électromagnétique de longueur d'onde comprise entre 0,8 et 2µm. Plus précisément, elle concerne un nouveau système formé d'un polymère amorphe possédant dans sa chaîne latérale un groupement photoréticulable furylacrylate, éloigné de la chaîne principale par un groupement intermédiaire, et d'une petite molécule comprenant un groupement chromophore sur lequel sont attachés au moins deux groupements photoréticulables furylacrylates. Le groupement chromophore, orienté au moyen d'un champ électrique continu donne naissance à des effets non linéaires du second ordre en optique. Le groupement réticulable sous l'action de la lumière ultraviolette permet de geler, au cours de la polarisation du matériau, l'orientation des entités responsables des effets quadratiques en optique. En effet, les matériaux polymères ont montré qu'ils permettaient d'obtenir des performances en optique non linéaire aussi satisfaisantes que les matériaux inorganiques type niobate de lithium ou arséniure de gallium ou dihydrogénophosphate de potassium et offrent en outre de grands avantages de mise en oeuvre, de coût et de variété de structure grâce à l'ingénierie moléculaire. Cependant, le manque de stabilité dans le temps des propriétés non linéaires d'ordre deux mesurées sur les films polymères reste un problème majeur posé par ces matériaux.

Des études ont montré que la création par réticulation, d'un réseau dense formé à partir des chromophores orientés sous champ, de telle sorte que l'entité active en optique non linéaire constitue véritablement un noeud ou un maillon du réseau, est un moyen particulièrement efficace pour stabiliser les propriétés optiques non linéaires d'ordre deux de ces matériaux.

Concernant l'énergie mise en jeu pour la formation du réseau, elle est, majoritairement, soit de nature thermique, soit de nature photochimique, mettant en jeu des mécanismes réactionnels très différents. D'une manière générale, les systèmes proposés ont été basés sur des réactions chimiques n'entraînant pas la formation de sous-produits volatiles, de façon à préserver les qualités optiques des films polymères lors de la réaction.

Les premiers réseaux polymères actifs en optique non linéaire de ce type, apparus dans la littérature, ont été obtenus par réticulation thermique.

Eich et Coll. ont été les premiers à vérifier expérimentalement l'augmentation de stabilité due à la réticulation (stabilité de plusieurs centaines d'heures à température ambiante), en formant un réseau dense de type époxy-amine à partir de diglycidyléther de bisphénol A (Bis-A), monomère bifonctionnel, et de 4-Nitro-1,2-Phenylenediamine (NPDA), monomère tétrafonctionnel (M. Eich et al., J. Appl. Phys., 66 7 (1989)). Ce système souffre cependant d'une concentration limitée en chromophores, liée à l'encombrement stérique important du Bis-A, ainsi que de performances mécaniques médiocres (film cassant) dues à une trop grande densification du réseau. Ils ont par la suite remédié à ces défauts, d'une part en remplaçant le Bis-A par du N,N-(diglycidyl)-4-Nitroaniline, ce qui a pour effet d'augmenter la teneur en chromophores, d'autre part en limitant la fixation des chromophores au seul groupement donneur, remplaçant à cette fin le NPDA par du N-(2-aminophenyl)-4-nitroaniline (NAN), ce qui conduit à un réseau moins contraint (D. Jungbauer et al., Appl. Phys. Lett., 56 26 (1990)). Dans ces conditions, des coefficients non linéaires d'ordre deux élevés ont été obtenus (d33 = 42 pm/V à 1064 nm), avec un maintien de 80% du signal pendant au moins 15 jours à 80°C. Cependant, ils se sont limités dans cette étude à des chromophores peu complexes, de longueur relativement faible, ce qui favorise probablement la stabilité.

Dans des approches plus récentes, l'entité active en optique non linéaire est plus complexe, de taille plus importante et constitue véritablement un noeud ou un maillon du réseau. Dans ce cas, elle est reliée par au moins deux liaisons covalentes avec le réseau, l'une d'entre elles se faisant à une des extrémités du chromophore, l'autre ou les autres s'opérant à l'autre extrémité.

Des exemples d'une telle réalisation sont donnés par l'élaboration d'un réseau polyuréthane à partir de petites molécules dihydroxylées dissoutes dans un polymère porteur de fonctions isocyanates (L. T. Cheng, R. P. Foss, G. R. Meredith, W. Tam, and F. C. Zumsteg, MRS Fall Meeting 1991, Boston), ou encore par la constitution d'un réseau de type époxy-amine, formé soit à partir de petites molécules actives en ONL fonctionnalisées à leur extrémité par des fonctions époxy, soit à partir de polymères à chaînes latérales actives en optique non linéaire, portant, à l'extrémité du chromophore, des fonctions réticulables par voie thermique (époxy ou amine) (S. Muller et al., brevet 90 13041), susceptibles de réagir, sous l'action de la chaleur, avec d'autres molécules polyfonctionnelles porteuses de groupements réticulables complémentaires.

Cependant, il faut noter que la formation de films polyuréthanes nécessite de travailler dans des conditions hygrométriques très strictes et que la formation des réseaux de type époxy-amine nécessite des temps de cuisson relativement importants.

Une deuxième source importante d'énergie pour la constitution d'un tel réseau est l'énergie photochimique. La réticulation par voie photochimique est généralement plus rapide que la réticulation par voie thermique. De plus, elle offre la possibilité de travailler à température ambiante, ce qui présente deux intérêts majeurs: le premier est de permettre des taux d'orientation plus élevés des chromophores, lié au fait que le facteur d'orientation des chromophores est inversement proportionnel à la température de polarisation., le second est de préserver le matériau d'une éventuelle dégradation thermique. Ces différents avantages ont pour conséquence une mise en oeuvre du matériau particulièrement aisée.

En outre, la réticulation photochimique pourrait permettre l'obtention de domaines alternés nécessaires à l'accord de phase, dans une application doublage de fréquence.

Des exemples de tels matériaux ont également été donnés dans le brevet déjà cité (S. Muller et al., brevet 90 13041).Dans ce cas, les polymères à chaînes latérales actives en optique non linéaire portent, à l'extrémité du chromophore, des fonctions réticulables photochimiquement susceptibles de réagir, sous l'action d'un rayonnement ultraviolet, avec d'autres molécules polyfonctionnelles porteuses des mêmes groupements réticulables photochimiquement.

Cependant, dans ce cas, l'orientation et le positionnement des groupements photoréticulables est imposé par l'orientation des chromophores, ce qui ne facilite pas la dimérisation des furylacrylates.

C'est pourquoi la présente invention propose une famille de matériaux composites formés d'un polymère porteur de fonctions photoréticulables particulières de type furylacrylate et d'une petite molécule active en optique non linéaire et possédant au moins deux fonctions photoréticulables furylacrylates.

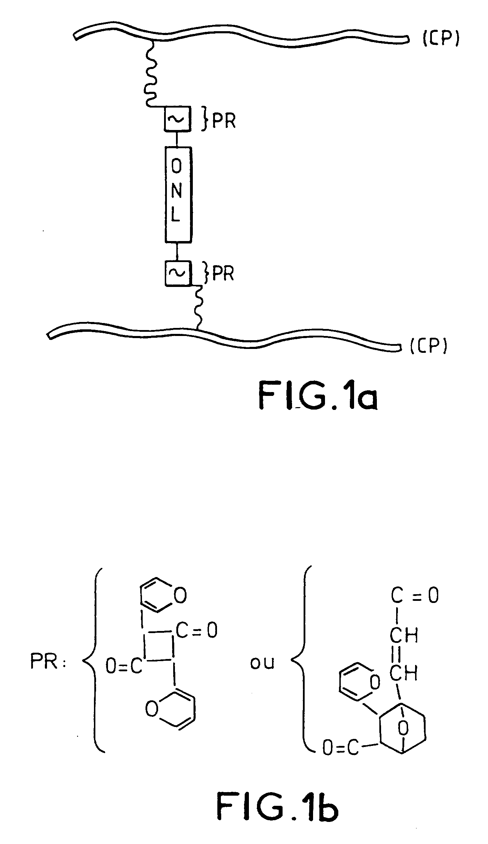

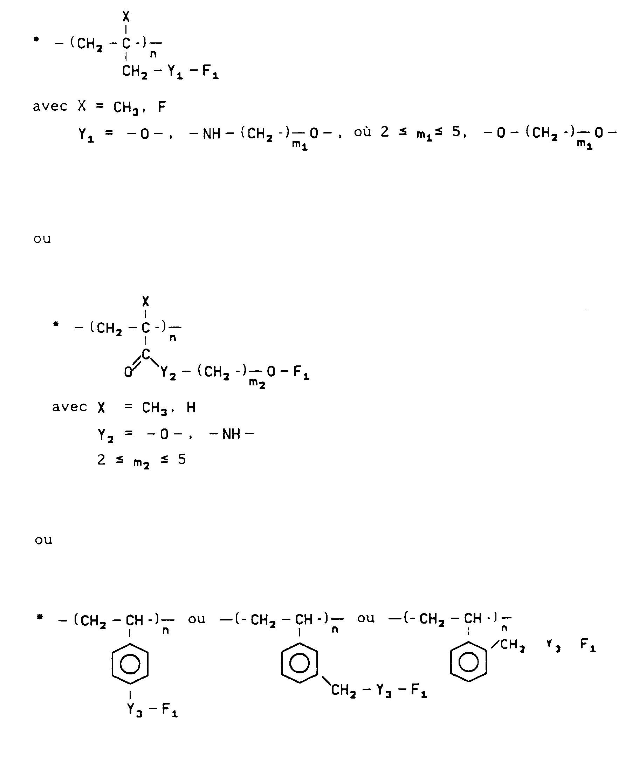

L'utilisation du furylacrylate est particulièrement intéressante en raison de la grande photosensibilité dudit groupement mais également en raison des possibilités de photodimérisation et de photopolymérisation (addition de la double liaison d'un groupement furylacrylate par réaction Diels-Alder) . La possibilité d'avoir ces deux réactions augmentent la probabilité de réaction entre deux groupements voisins. Ce comportement est illustré à la figure la sur laquelle sont représentées les chaînes principales du polymère (CP), l'association (PR) de deux groupements photoréticulables ; la figure 1b illustre les configurations possibles pour l'association PR et le groupement ONL représente la partie active de la petite molécule.

Les matériaux selon l'invention peuvent ainsi après réticulation être particulièrement stables dans le temps. La nature de la petite molécule est adaptée aux applications visées, soumises aux exigences de transparence à la longueur d'onde d'application visée tout en offrant une structure la plus active possible en optique non linéaire.

Plus précisément, l'invention a pour objet un matériau composite utilisable en optique non linéaire comprenant :
- un polymère amorphe constitué d'une chaîne principale sur laquelle sont rattachées des chaînes latérales ;
- une petite molécule capable de générer des effets en optique non linéaire ;
caractérisé en ce qu'une chaîne latérale possède un groupement photoréticulable furylacrylate 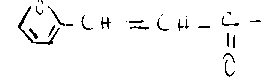 éloigné du squelette du polymère par un groupement intermédiaire et que la petite molécule comprend également aux moins deux groupements furylacrylate.

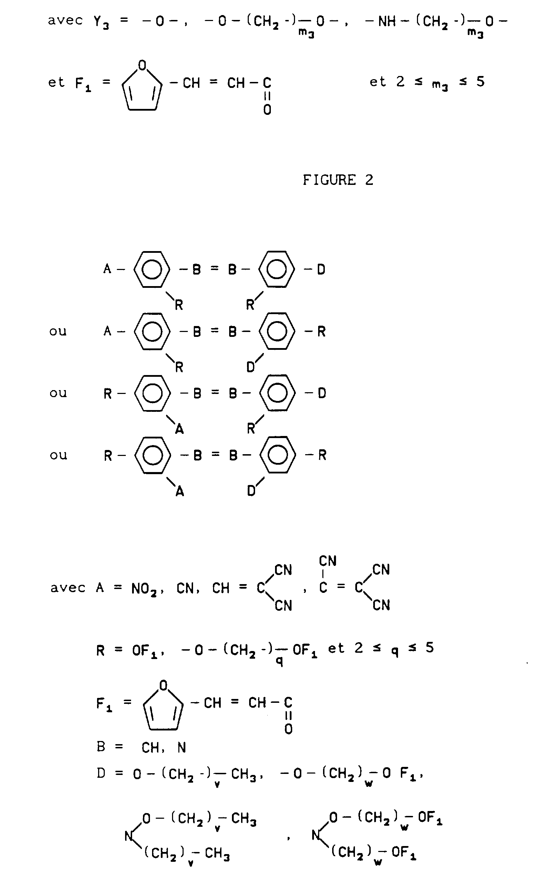

De préférence, le polymère amorphe répond à l'une des formules illustrée à la figure 2.

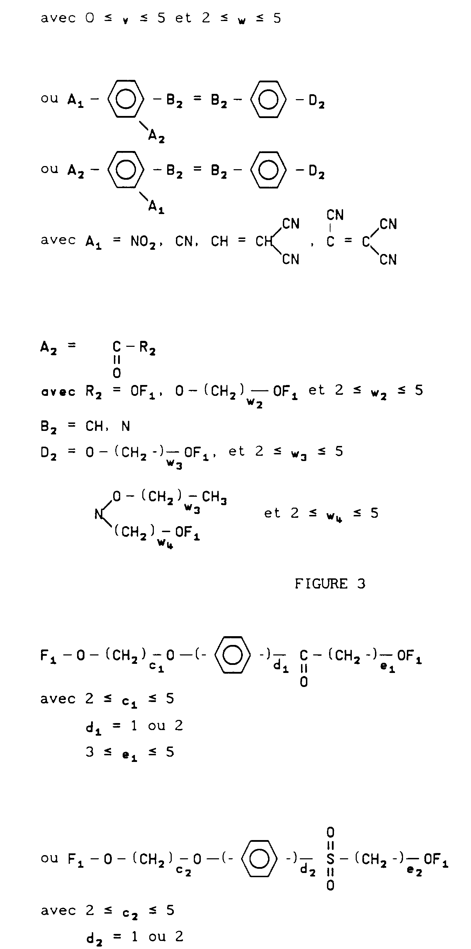

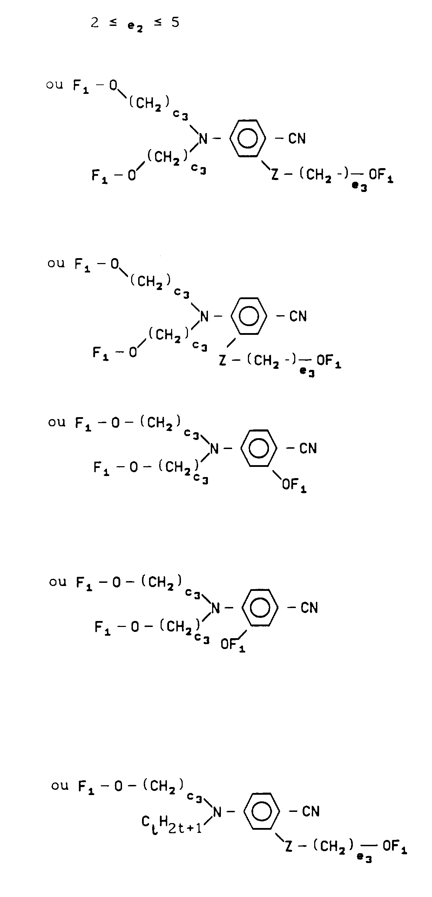

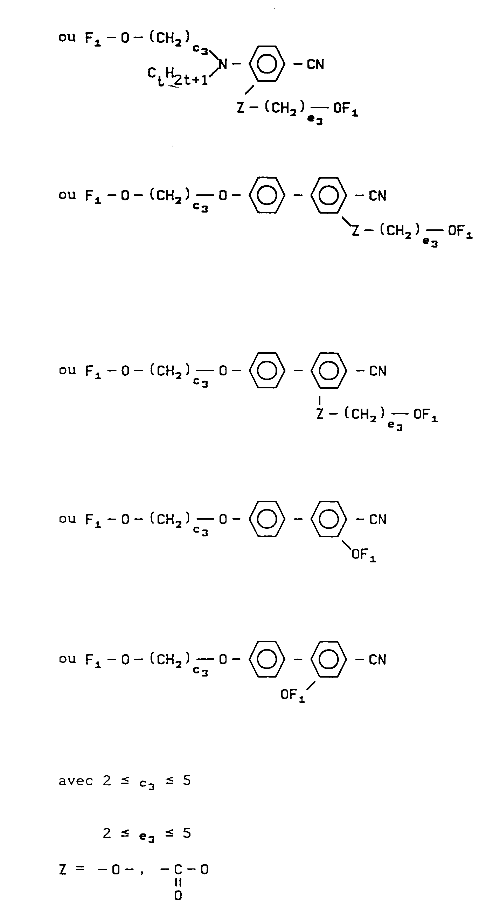

Dans le cadre d'application concernant les télécommunications qui ont choisi comme longueur d'onde 1,32 µm, la petite molécule choisie peut avantageusement être transparente dans la gamme 0,8 µm-2µm et répondre à l'une des formules chimiques illustrées à la figure 3.

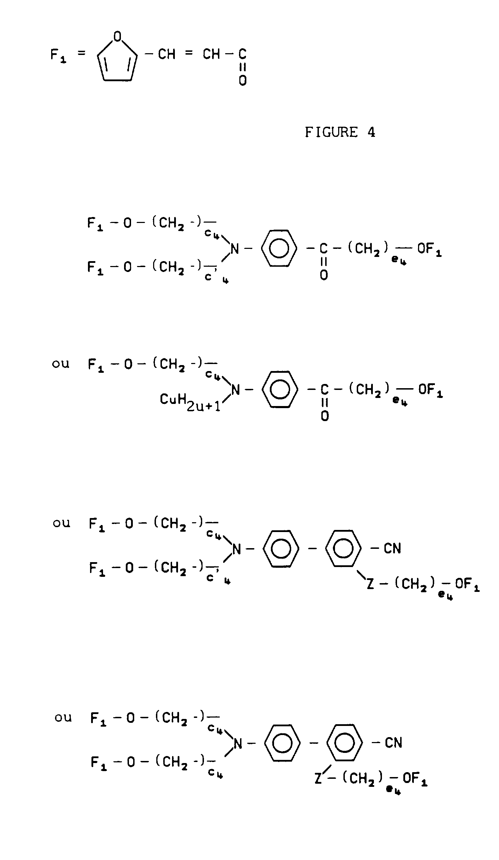

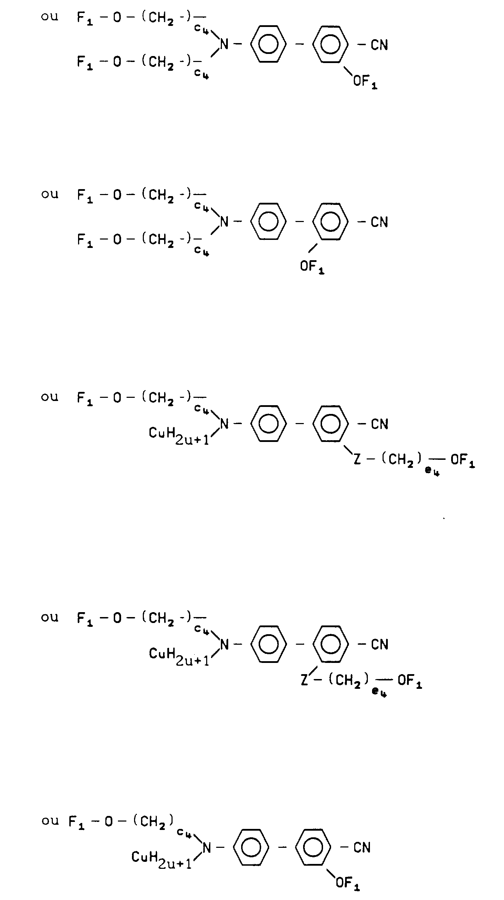

Pour la réalisation de doubleur de fréquence en vue de l'obtention de source laser bleue, la petite molécule peut avantageusement répondre à l'une des formules chimiques suivantes illustrées à la figure 4.

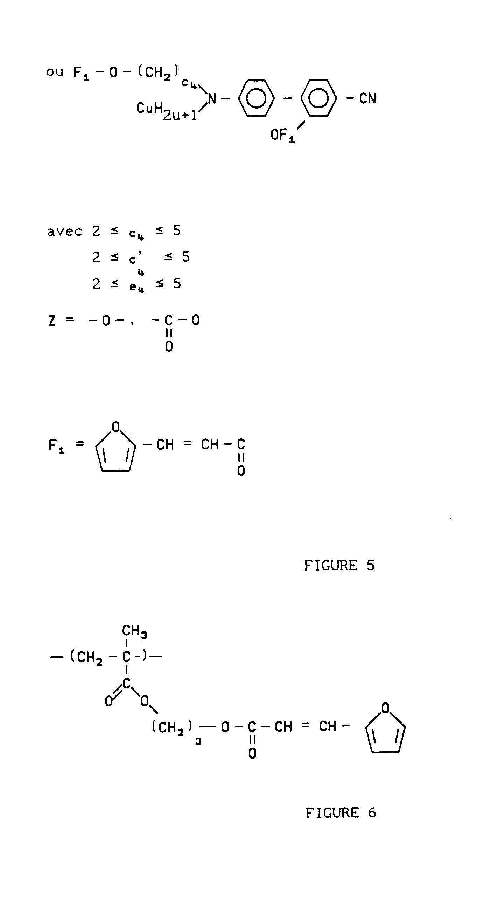

Pour la réalisation de doubleur de fréquence en vue de l'obtention de source laser verte, la petite molécule peut avantageusement répondre à l'une des formules chimiques suivantes illustrées à la figure 5.

L'invention a également pour objet un procédé de synthèse du polymethacryloyloxypropyl furylacrylate ainsi qu'un procédé de synthèse du 2,2' bis (furylacryloxy) 4 diethylamino 4' nitroazobenzène.

L'invention sera mieux comprise et d'autres avantages apparaîtront à la lecture de la description qui va suivre donnée à titre non limitatif et grâce aux figures annexées parmi lesquelles:
- La figure 1 représente la structure du matériau réticulé selon l'invention:
   . la figure 1a illustre la structure polymère, petite molécule après réticulation ;
   . la figure 1b schématise l'association de deux groupements photoréticulables.
- La figure 2 représente la structure moléculaire des homopolymères hôtes selon l'invention ;
- La figure 3 représente la structure chimique des petites molécules actives en optique non linéaire photoréticulables pour application au modulateur électrooptique ;
- La figure 4 représente la structure chimique des petites molécules actives en optique non linéaire photoréticulables pour application au doubleur de fréquence en vue de l'obtention de source bleue ;
- La figure 5 représente la structure chimique de petites molécules actives en optique non linéaire photoréticulables pour application au doubleur de fréquence en vue de l'obtention de source verte ;
- La figure 6 représente la structure chimique d'un exemple d'homopolymère selon l'invention ;
- La figure 7 représente la structure chimique d'un exemple de petite molécule active en optique non linéaire photoréticulable selon l'invention ;
- La figure 8 représente le schéma réactionnel de synthèse de l'exemple d'homopolymère illustré à la figure 6 ;
- La figure 9 représente le schéma réactionnel de synthèse de l'exemple de la petite molécule photoréticulable illustrée à la figure 7 ;
- La figure 10 donne l'évolution de la densité optique d'un système polymère petite molécule selon l'invention en fonction du temps d'irradion à 312 nm.

### EXEMPLE

L'exemple concerne la synthèse du poly(méthacryloyloxypropylfurylacrylate) et du 2,2'-bis(furylacryloxy) 4 diéthylamino 4' nitroazobenzène

Synthèse d'un homopolymère représenté à la figure 6.

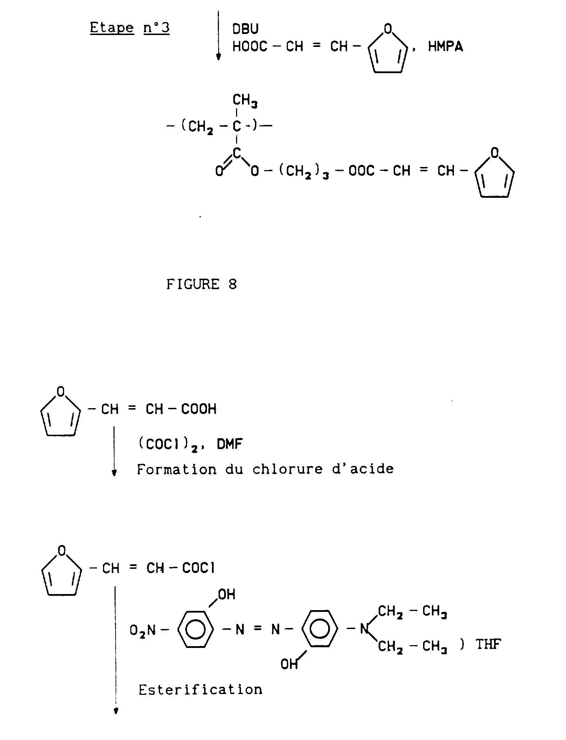

L 'homopolymère est préparé en 3 étapes à partir du chlorure de méthacryloyle et du 3 bromo propanol commerciaux selon le schéma réactionnel de la figure 8.

### Première étape: synthèse du méthacrylate de 3 bromopropanol

Dans un erlenmeyer de 11 muni d'une agitation magnétique, sont versés 27,9 g de 3 bromopropanol dans 200 ml d'éther. A cette solution est versée goutte à goutte une solution de 20,9 g de chlorure de méthacryloyle dans 200 ml d'éther. Au mélange précédent est additionné goutte à goutte 26,3 g de triéthylamine en solution dans un peu d'éther. La réaction est menée toute une nuit. L'avancement de la réaction est contrôlé par prélèvement d'un peu du milieu réactionnel, évaporation de l'éther et analyse en IR. Le sel de bromhydrate de triéthylamine est éliminé par filtration, puis la solution éthérée est lavée par une solution aqueuse d'acide chlorhydrique à PH1. Après plusieurs lavages à l'eau, la phase éthérée est séchée sur sulfate de magnésium qui est éliminé ensuite par filtration, puis évaporée au rotavapor. Le produit est purifié par deux distillations successives (Eb (1mm Hg) = 64°C.

### Deuxième étape: Homopolymérisation du méthacrylate de 3 bromopropanol(monomère)

Dans l'ampoule de polymérisation sont successivement introduits: 19,8 mg d'azobisisobutyronitrile, 5g de monomère, 50 ml de diméthylformamide; Après trois dégazages sous vide poussé, l'ampoule de polymérisation est scellée. La réaction est menée 24 h00 à 60°C. La solution est ensuite diluée dans le même volume de dichlorométhane et versée goutte à goutte dans 1,51 d'éthanol sous forte agitation. Le précipité blanc obtenu est filtré et séché sous vide à 40°C pendant une nuit. Le rendement obtenu est de 80%.

### Troisième étape: Synthèse du poly(méthacryloyloxypropylfurylacrylate) (polymère)

La synthèse est réalisée par greffage de l'acide furylacrylique sur le polymère. Celui-ci est dissout dans 35 ml d'héxaméthyphosphoretriamide (HMPA) à 40 °C. A cette solution est ajoutée goutte à goutte une solution de 1,03 g d'acide furylacrylique dans 40 ml d' HMPA. 1,15 g de 1,8-diazabicyclo (5.4.0)-Undec-7-ène (DBU) sont ensuite introduits dans 20 ml d' HMPA. La réaction est menée toute une nuit à 40°C.

La solution ainsi obtenue est versée goutte à goutte dans 1,6 l d'eau sous agitation. Un précipité beige apparaît. Il est isolé par filtration et mis à sécher au dessicateur à 50°C. Rendement: 90%

Le polymère obtenu présente un taux de greffage, évalué d'après la RMN, de 87% et présente une température de transition vitreuse, mesurée par analyse enthalpique différentielle , de 40°C.

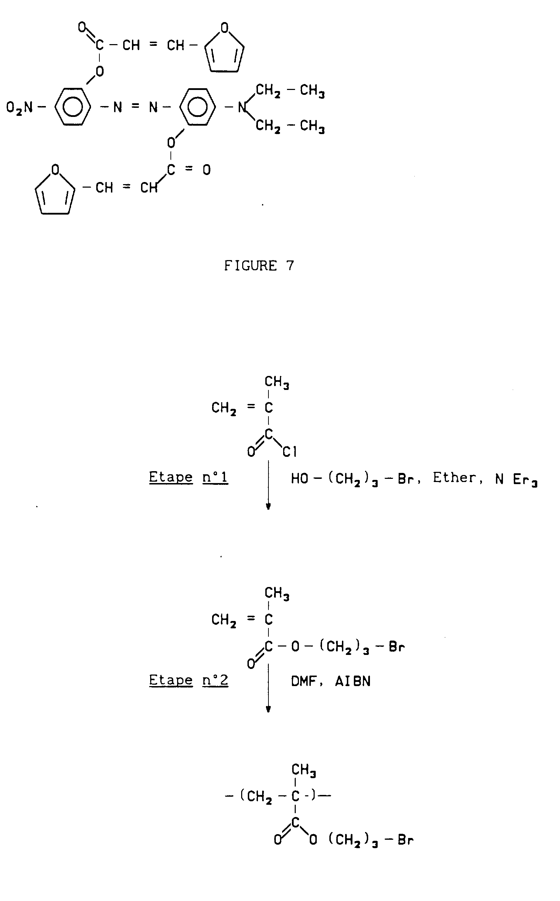

Synthèse d'une petite molécule selon l'invention représentée à la figure 7.

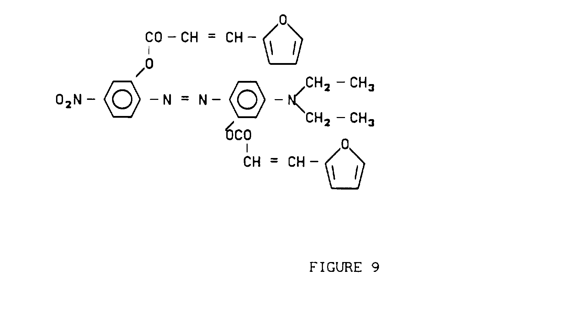

La molécule active en optique non linéaire et photoréticulable est obtenue en 2 étapes. La deuxième étape s'effectue à partir du 2,2'-bis(hydroxy) 4 diéthylamino 4'nitroazobenzène, qui s'obtient par une réaction de diazotation bien connue de l'homme de l'art ; le schéma réactionnel étant représenté à la figure 9.

### Première étape: Synthèse du chlorure de furylacryloyle.

Dans un ballon de 50 ml muni d'une agitation magnétique sont introduits 3,35 g d'acide furylacrylique et 10, 2 g de chlorure d'oxalyle. On agite et on ajoute 6 gouttes de DMF. Le mélange est agité 1H00 à température ambiante, puis l'excès de chlorure est évaporé. La formation du chlorure est vérifiée par spectroscopie Infra rouge. Il est utilisé, pour l'étape suivante, immédiatement après sa formation.

### Deuxième étape: Synthèse du 2,2'-bis(hydroxy) 4 diéthylamino 4' nitroazobenzène(diol)

4,17 g de chlorure de furylacryloyle précédemment obtenu, en solution dans 6ml de tétrahydrofurane sont additionnés goutte à goutte à une solution de 2g 2,2'-bis (hydroxy)4 diethylamino 4' nitroazobenzène et 1,92g de triéthylamine dans 6 ml de tétrahydrofurane refroidis à 5°c (bain eau + glace).

Le mélange est agité une nuit à température ambiante . Le solvant est ensuite évaporé sous vide . Le résidu est solubilisé dans le dichlorométhane, lavé à l'eau, puis séché au sulfate de magnésium. Le produit est ensuite purifié par Flash-chromatographie avec le chloroforme comme éluant. Après purification par recristallisation dans un mélange hexane/chloroforme, le rendement obtenu est de 50%. Le produit présente une absorption maximale à 505 nm. Point de fusion: 175,5 °C.

La structure des produits obtenus à chaque étape de la synthèse est vérifiée par spectroscopies IR et RMN 1H 250MHz.

### OBTENTION DE FILMS ORIENTES ET RETICULES.

On réalise une solution de polymère-hôte et de petite molécule active en optique non linéaire de telle manière que la concentration en chaînes latérales du polymère porteuses des fonctions phoréticulables soit égale à au moins 2 fois celle du chromophore. Le mélange est déposé par centrifugation sur un substrat conducteur ou semiconducteur. Le film obtenu est séché sous vide. Après élimination du solvant d'enduction, le film est polarisé par application d'un champ électrique au moyen d'une pointe Corona portée à un potentiel supérieur ou égal à 4kV. Parallèlement le film est irradié au moyen d'une lampe ou d'un ensemble de lampes émettant dans la bande d'absorption du groupement photoréticulable pendant le temps nécessaire à la photoréticulation. Ce temps ayant été déterminé au préalable par une étude spectrométrique.

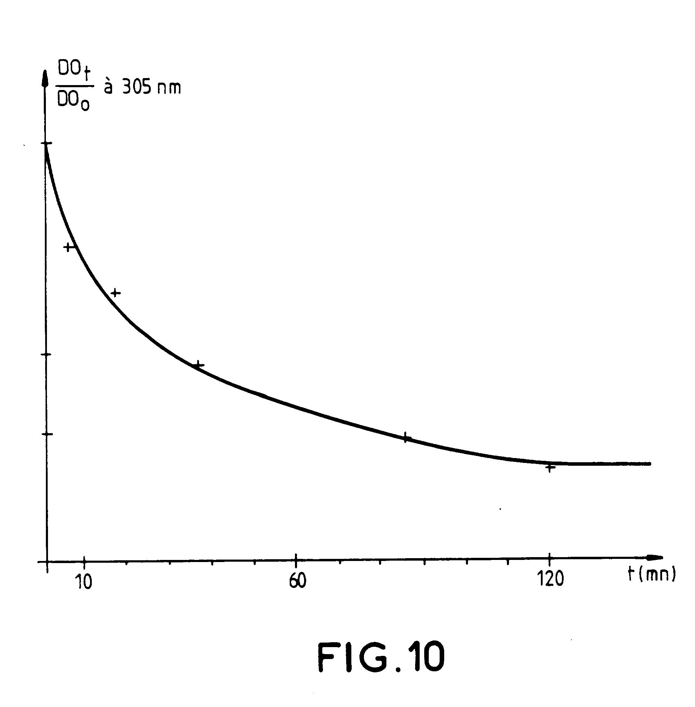

A titre d'exemple une solution contenant
0,1g de poly(méthacryloyloxypropylfurylacrylate)
0,02g de 2,2'-bis(furylacryloxy) 4 diéthylamino 4'nitroazobenzène
2 ml de dioxane
est tout d'abord déposée par centrifugation ( 2500 tours par minute pendant 30 secondes) sur un substrat en quartz, lui-même recouvert d'un autre substrat de quartz, afin de réaliser l'étude spectrométrique. Le film ayant un maximum d'absorption à 305 nm, relatif aux groupements furylacrylates, une lampe émettant à 312nm est choisie pour effectuer la photoréticulation. La figure 10 montre l'évolution de la densité optique mesurée à 305 nm, en fonction du temps. On constate une rapide décroissance de la densité optique dans les 30 premières minutes d'irradiation en accord avec la réticulation du système. On peut estimer que l' état d'avancement de la réaction est suffisant au bout de 1H25. Ceci est par ailleurs vérifié en immergeant le film dans le dioxane afin d'éliminer les éventuelles petites molécules non engagées dans le réseau. Aucune coloration du solvant n'est observée, témoignant ainsi de l'absence de chromophores. D'autre part, la densité optique du film mesurée par spectrométrie est en tous points semblable à celle obtenue avant immersion du film.

Compte-tenu de ces résultats il est maintenant possible d'obtenir un film réticulé et polarisé. Pour ce faire on dépose un nouveau film sur un substrat conducteur ou semiconducteur, dans les mêmes conditions que précédemment et on irradie à 312 nm en même temps qu'on applique un champ continu au moyen d'une pointe Corona portée à 4kV.

## Revendications

1. Matériau composite utilisable en optique non linéaire comprenant :
- un polymère amorphe constitué d'une chaîne principale sur laquelle sont rattachées des chaînés latérales ;
- une petite molécule capable de générer des effets en optique non linéaire ;
caractérisé en ce qu'une chaîne latérale possède un groupement photoréticulable furylacrylate 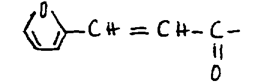 éloigné du squelette du polymère par un groupement intermédiaire et que la petite molécule comprend également aux moins deux groupements furylacrylate.

2. Matériau selon la revendication 1, caractérisé en ce que le polymère amorphe répond à l'une des formules chimiques suivantes :

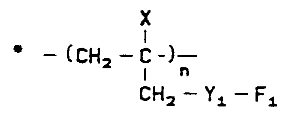

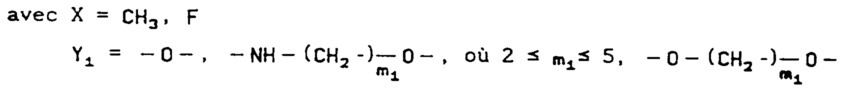

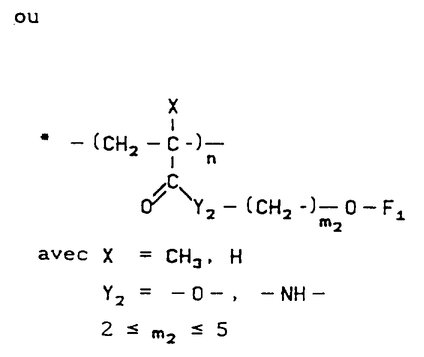

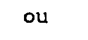

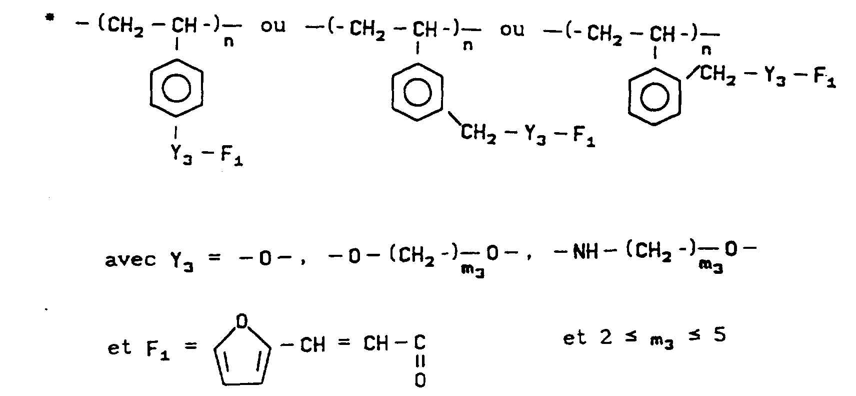

3. Matériau selon l'une des revendications 1 ou 2, caractérisé en ce que la petite molécule est transparente dans la gamme 0,8 µm-2 µm et répond à l'une des formules chimiques suivantes :

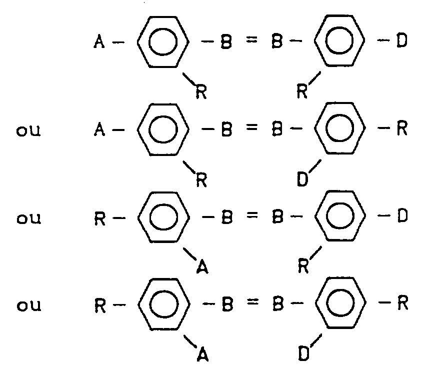

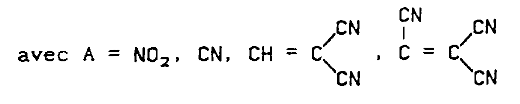

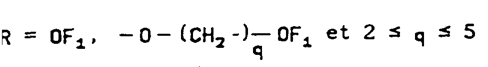

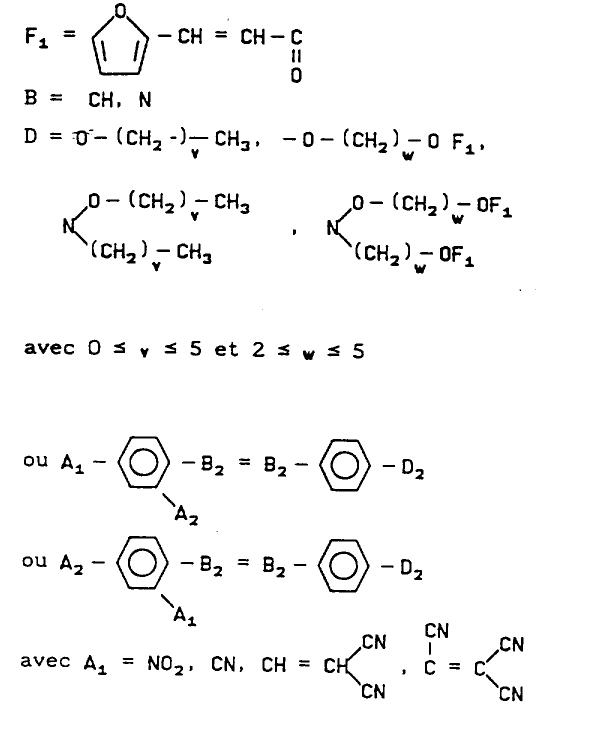

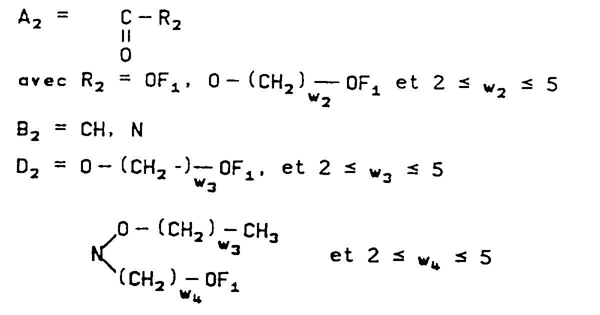

4. Matériau selon l'une des revendications 1 ou 2, caractérisé en ce que la petite molécule est transparente à des longueurs d'onde supérieures à 300 nm et qu'elle répond à l'une des formules chimiques suivantes :

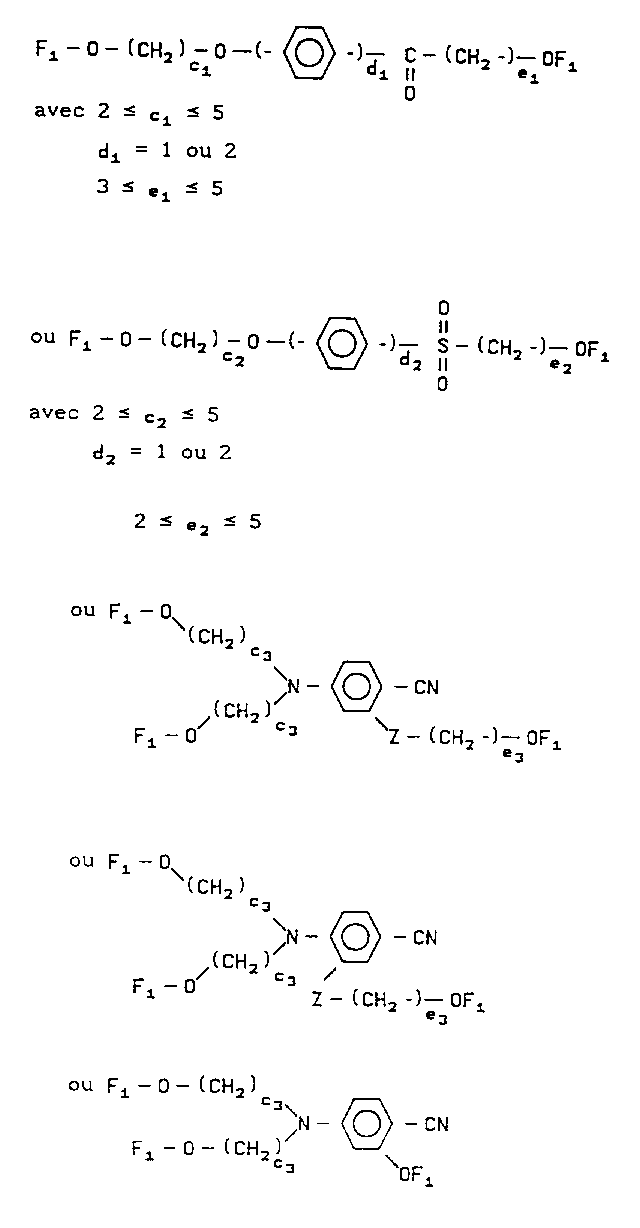

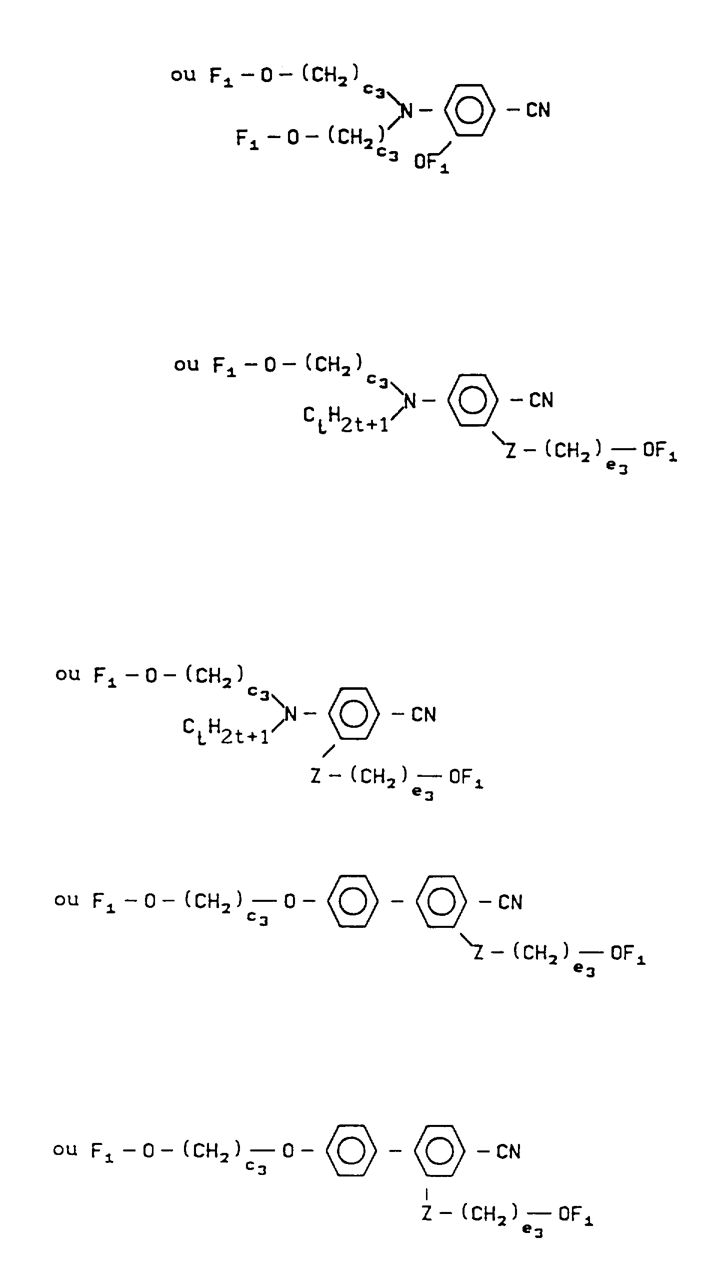

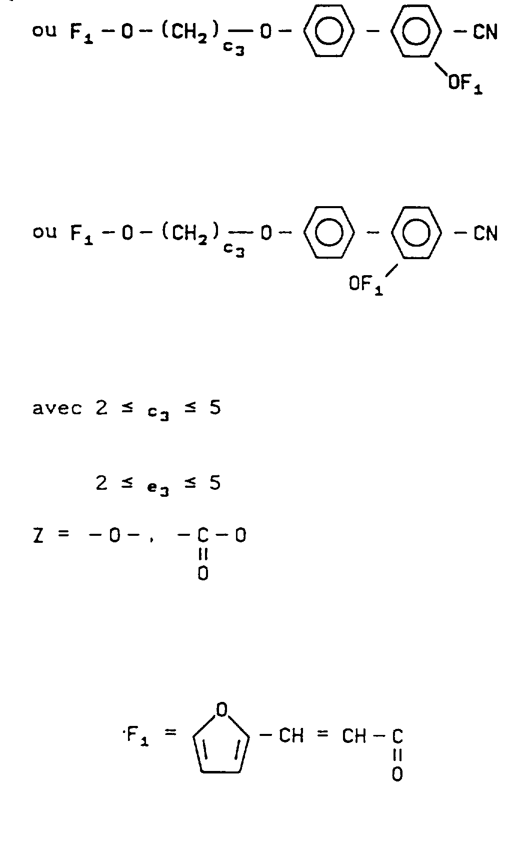

5. Matériau selon l'une des revendications 1 ou 2, caractérisé en ce que la petite molécule est transparente à des longueurs d'onde supérieures à 350 nm et qu'elle répond à l'une des formules chimiques suivantes :

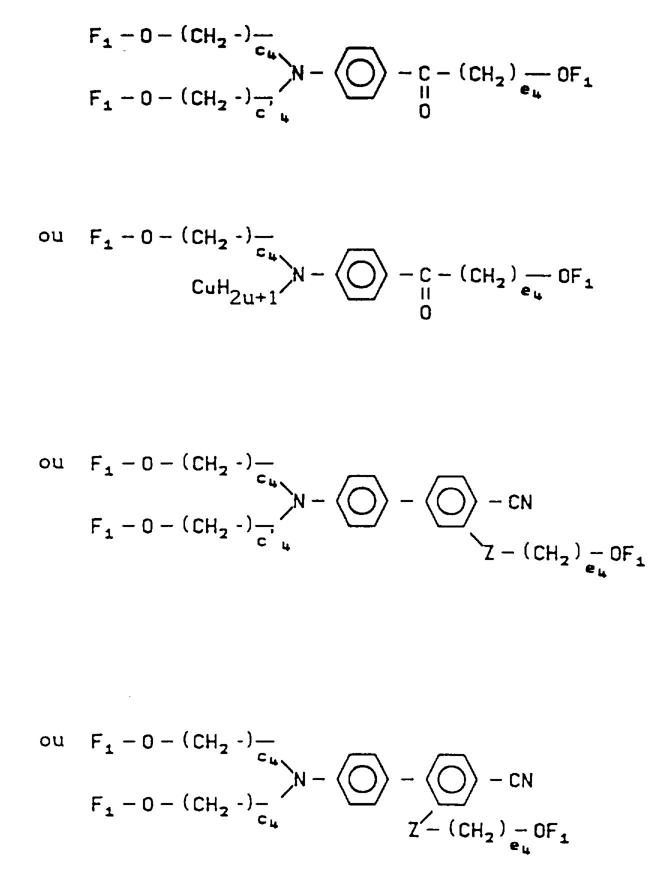

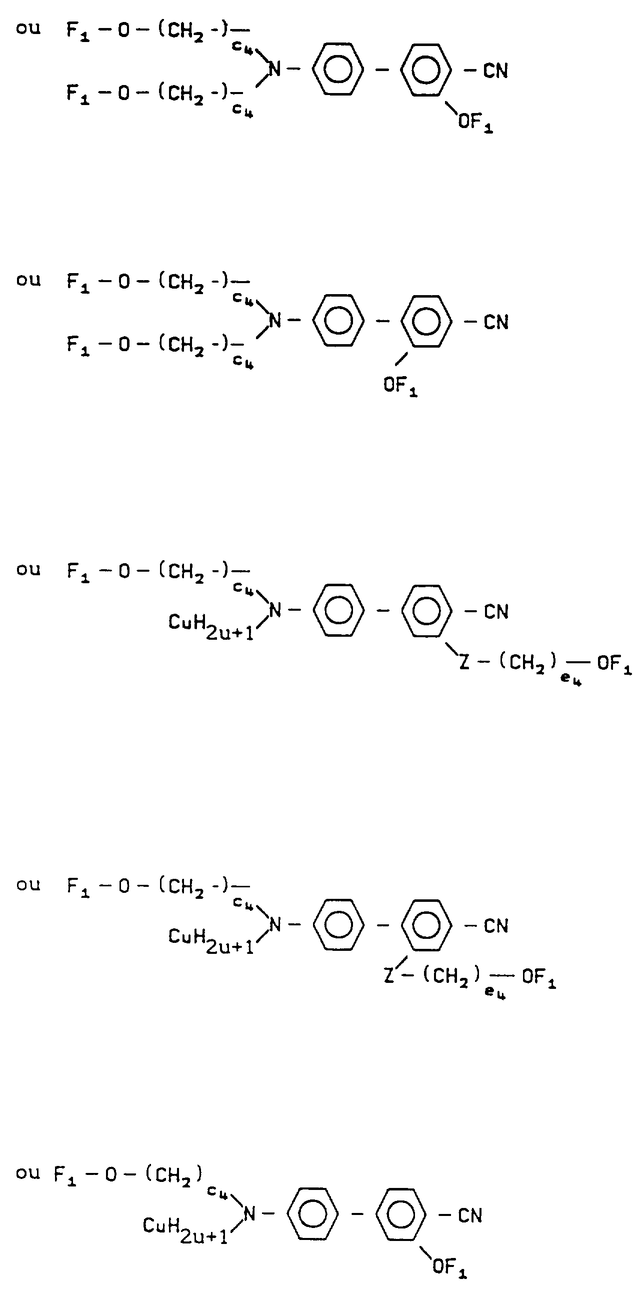

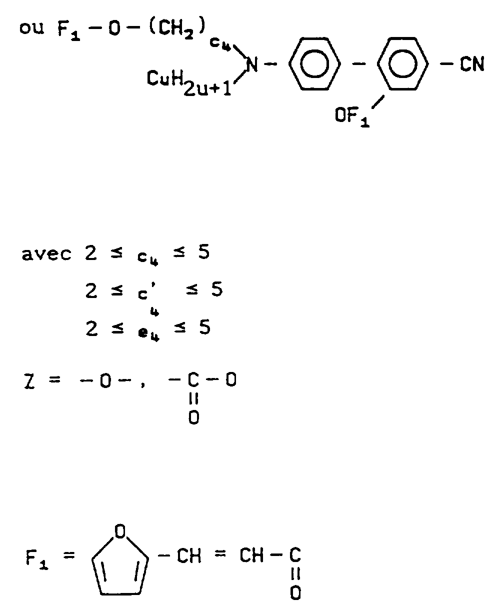

6. Procédé de synthèse d'un matériau composite utilisable en optique non linéaire selon la revendication 1 comprenant la synthèse du polyméthacryloyloxypropylfurylacrylate caractérisé en ce qu'il comporte les étapes suivantes :
1. Synthèse du méthacrylate de 3 bromopropanol à partir de l'esterification du chlorure de méthacryloyle en présence du 3 bromopropanol ;
2. Homopolymérisation radicalaire du méthacrylate de 3 bromopropanol en présence de diméthylformamide (DMF) et d'azobisisobutyronitrile (AIBN);
3. Esterification de l'homopolymère obtenu à l'étape 2, en présence d'hexaméthylphosphoretriamide (HMPA), de 1,8-diazabicyclo (5.4.0)-Undec-7-ène (DBU) et d'acide furylacrylique.

7. Procédé de synthèse d'un matériau composite utilisable en optique non linéaire selon la revendication 1 comprenant la synthèse du 2,2'-bis (furylacryloxy) 4 diéthylamino 4' nitroazobenzène caractérisé en ce qu'il comprend les étapes suivantes :
1. Synthèse du chlorure de furylacryloyle en présence d'acide furylacrylique, de chlorure d'oxalyte et de DMF ;
2. Esterification du chlorure de furylacryloyle en présence de tetrahydrofurane (THF) et du 2,2'-bis (hydroxy) 4 diéthylamino 4' nitroazobenzène.

## Patentansprüche

1. Zusammengesetztes Material zur Verwendung in der nichtlinearen Optik, umfassend:
- ein aus einer Hauptkette und an die Hauptkette gebundene Seitenketten gebildetes amorphes Polymer; und
- ein zur Erzeugung nichtlinear-optischer Effekte fähiges kleines Molekül, dadurch gekennzeichnet, daß eine Seitenkette eine photovernetzbare Furylacrylatgruppe 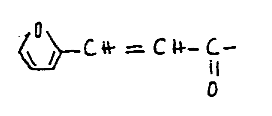
besitzt, die von dem Polymerskelett durch eine Zwischen-Gruppe auf Abstand gehalten ist, und daß das kleine Molekül gleichfalls wenigstens zwei Furylacrylatgruppen umfaßt.

2. Material nach Anspruch 1, dadurch gekennzeichnet, daß das amorphe Polymer einer der folgenden chemischen Formeln entspricht:

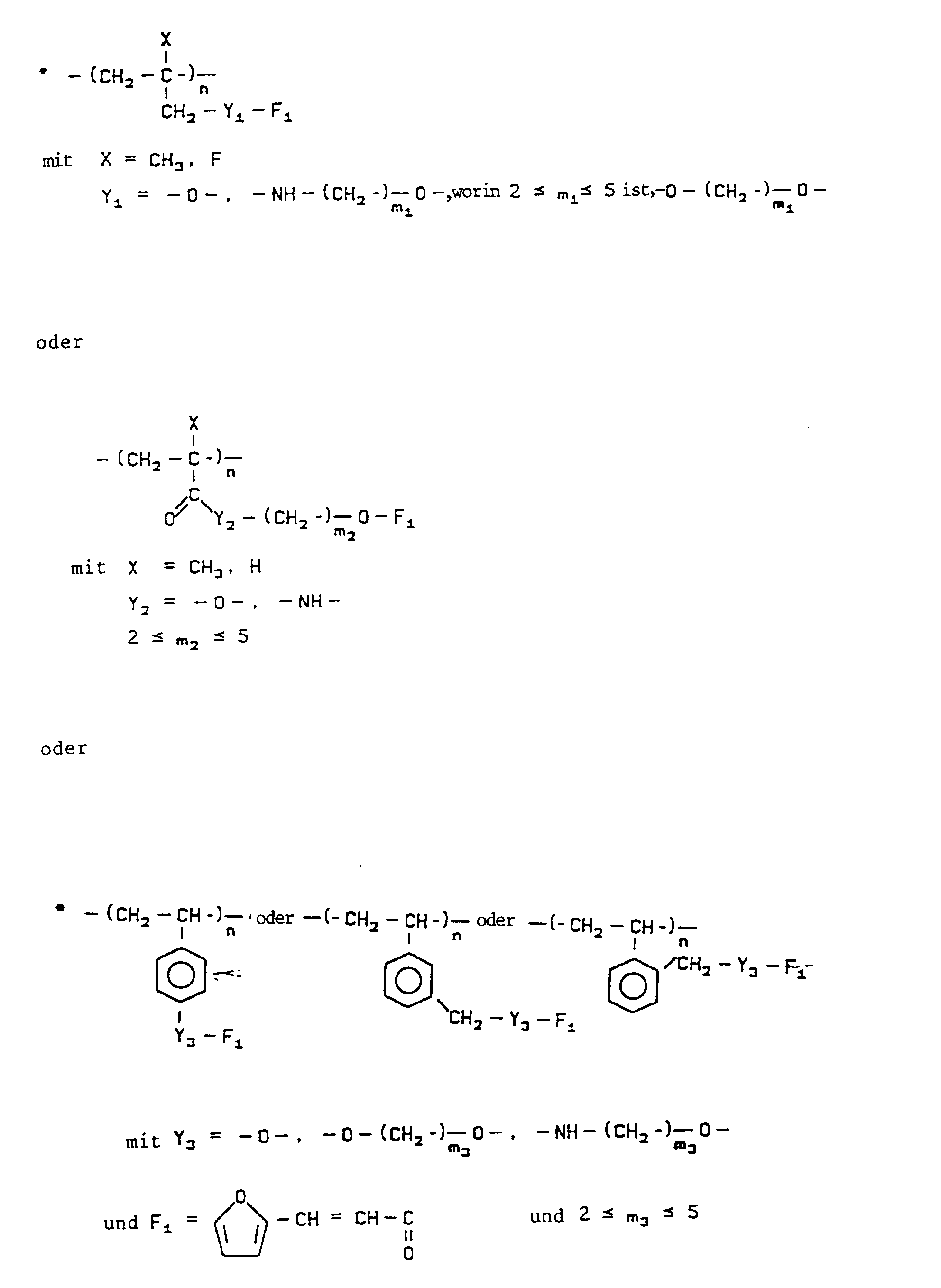

3. Material nach einem der Ansprüche 1 oder 2, dadurch gekennzeichnet, daß das kleine Molekül im Bereich von 0,8 µm - 2 µm transparent ist und einer der folgenden chemischen Formeln entspricht:

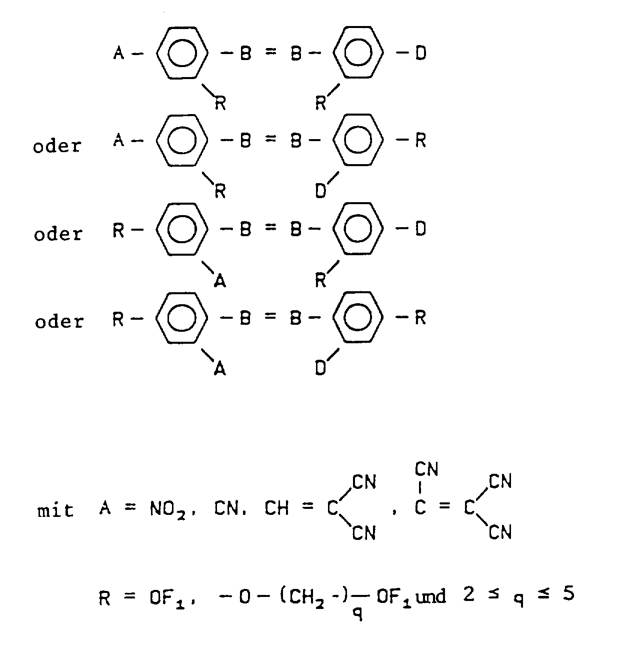

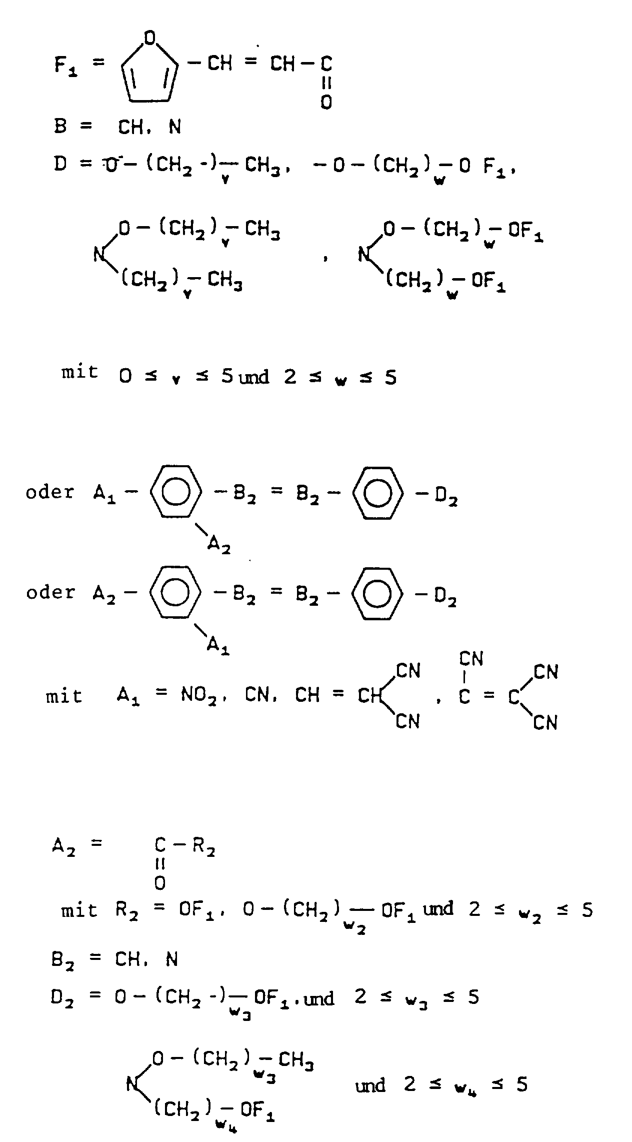

4. Material nach einem der Ansprüche 1 oder 2, dadurch gekennzeichnet, daß das kleine Molekül bei Wellenlängen oberhalb 300 nm transparent ist und einer der folgenden chemischen Formeln entspricht:

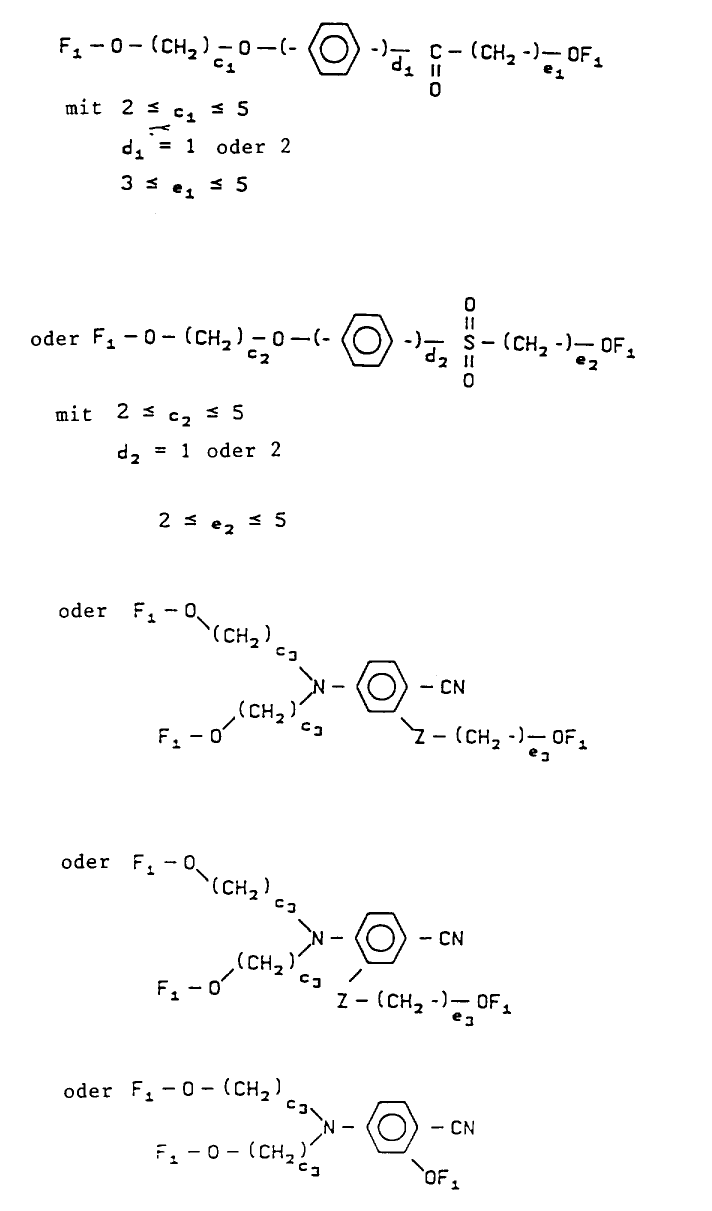

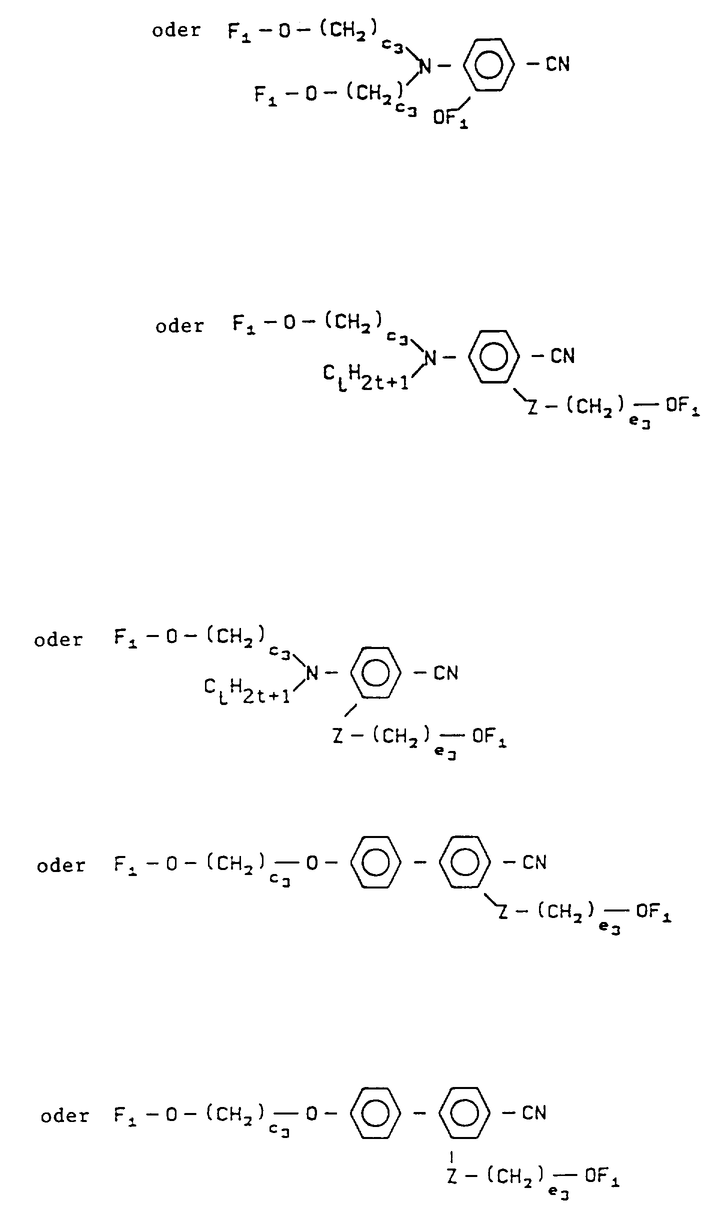

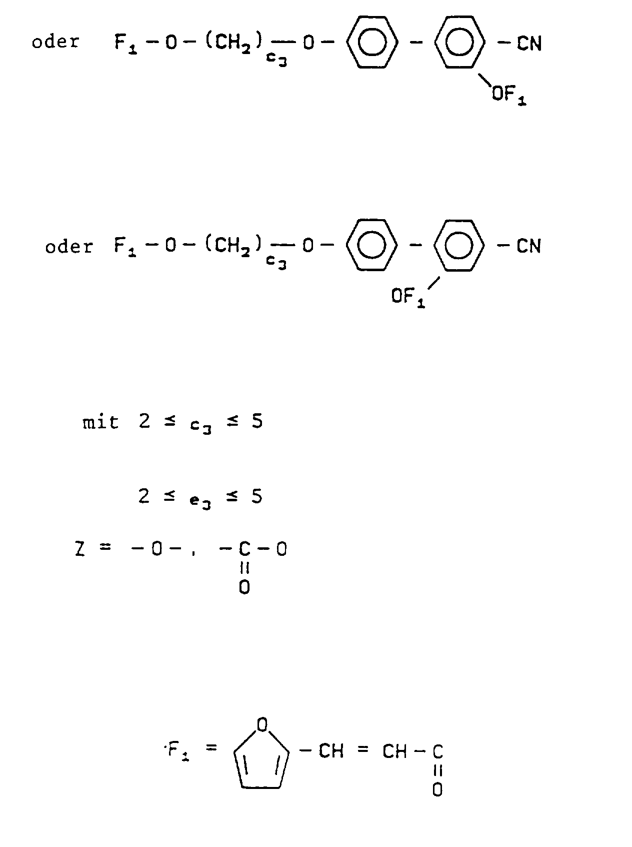

5. Material nach einem der Ansprüche 1 oder 2, dadurch gekennzeichnet, daß das kleine Molekül bei Wellenlängen oberhalb 350 nm transparent ist und einer der folgenden chemischen Formeln entspricht:

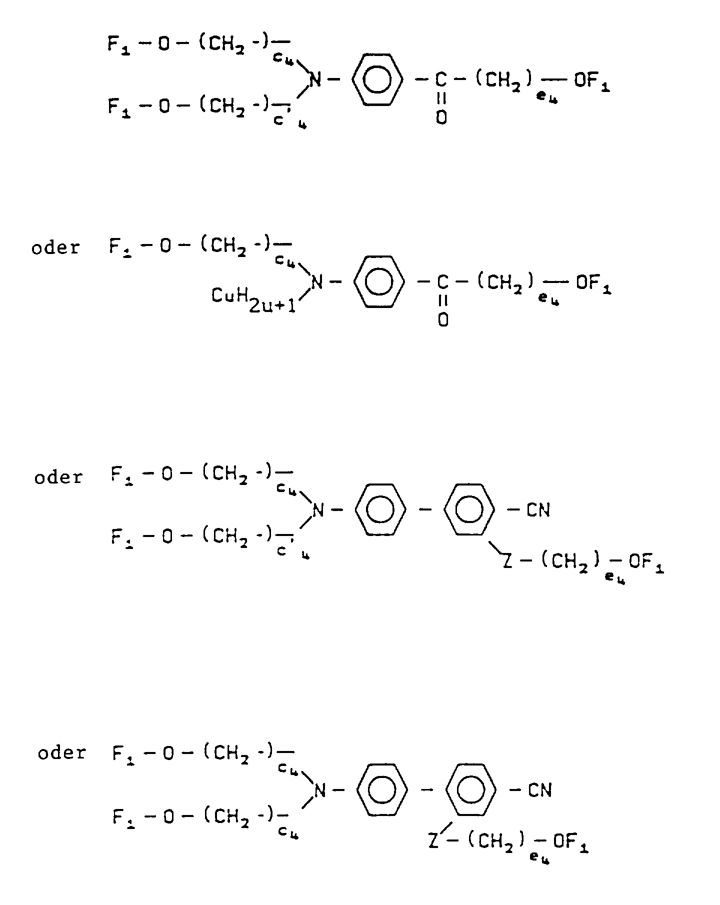

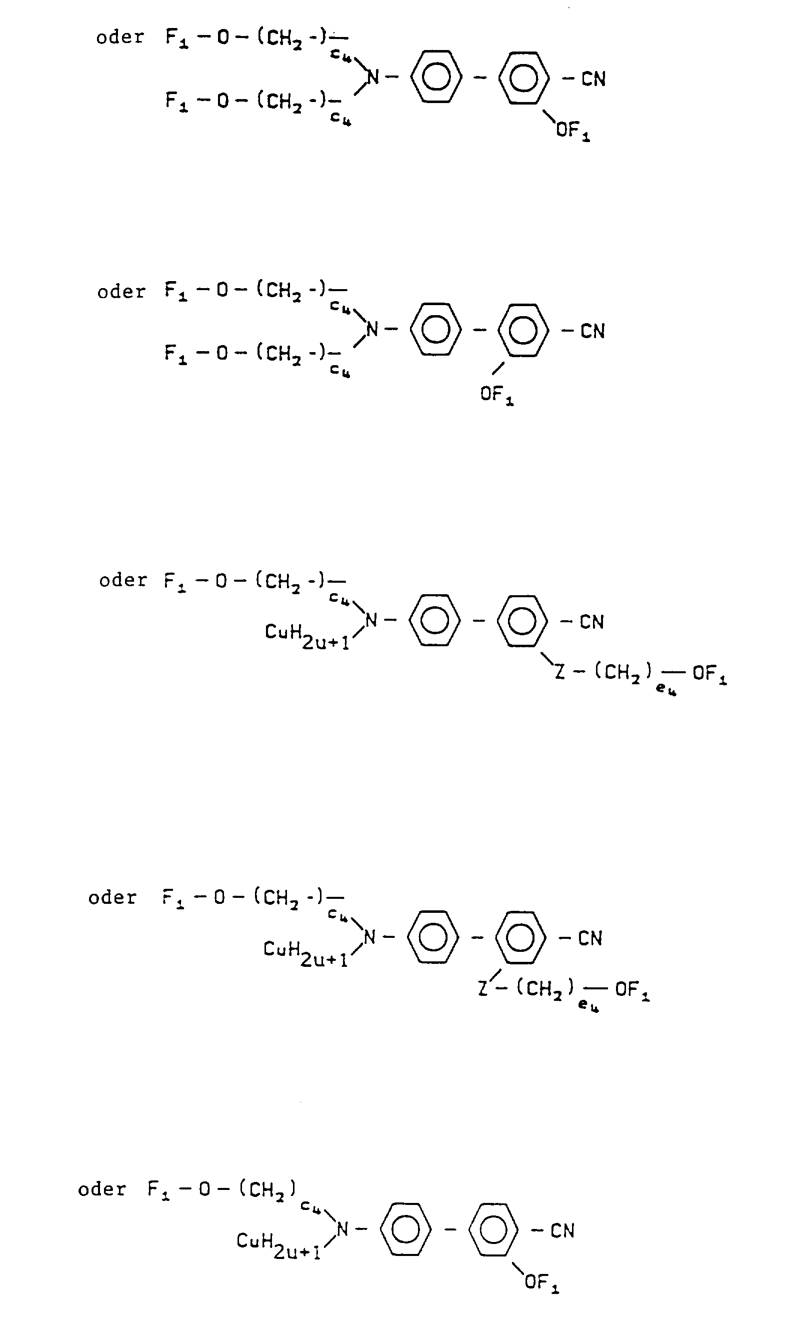

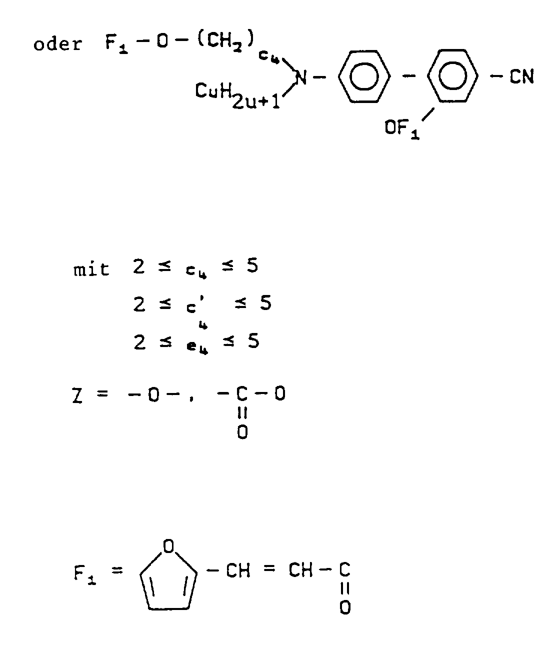

6. Verfahren zur Herstellung eines in der nichtlinearen Optik verwendbaren zusammengesetzten Materials gemäß Anspruch 1, welches die Herstellung von Polymethacryloyloxypropylfurylacrylat umfaßt, dadurch gekennzeichnet, daß es die folgenden Stufen beinhaltet:
1. Herstellung von 3-Brompropanmethacrylat durch Verestern von Methacrylsäurechlorid in Gegenwart von 3-Brompropanol;
2. radikalische Homopolymerisation von 3-Brompropanmethacrylat in Gegenwart von Dimethylformamid (DMF) und Azobisisobutyronitril (AIBN);
3. Veresterung des in Stufe 2 erhaltenen Homopolymers in Gegenwart von Hexamethylphosphorsäuretriamid (HMPA), 1,8-Diazabicyclo[5.4.0]Undec-7-en (DBU) und Furylacrylsäure.

7. Verfahren zur Herstellung eines in der nichtlinearen Optik verwendbaren zusammengesetzten Materials gemäß Anspruch 1, welches die Herstellung von 2,2'-Bis(furylacryloxy)-4-diethylamino-4'-nitroazobenzol umfaßt, dadurch gekennzeichnet, daß es die folgenden Stufen umfaßt:
1. Herstellung von Furylacrylsäurechlorid in Gegenwart von Furylacrylsäure, Oxalsäurechlorid und DMF;
2. Veresterung des Furylacrylsäurechlorids in Gegenwart von Tetrahydrofuran (THF) und 2,2'-Bis(hydroxy)-4-diethylamino-4'-nitroazobenzol.

## Claims

1. Composite material which can be used in non-linear optics comprising:
- an amorphous polymer composed of a main chain to which are attached side chains;
- a small molecule capable of generating non-linear optical effects;
characterized in that a side chain possesses a photocrosslinkable furylacrylate group 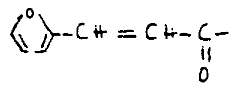 separated from the skeleton of the polymer by an intermediate group and in that the small molecule also comprises at least two furylacrylate groups.

2. Material according to Claim 1, characterized in that the amorphous polymer corresponds to one of the following chemical formulae: 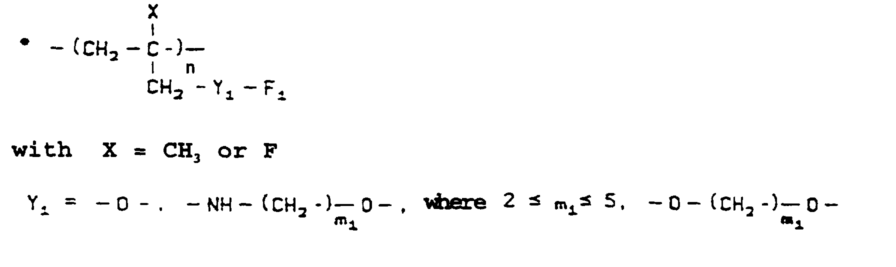 or 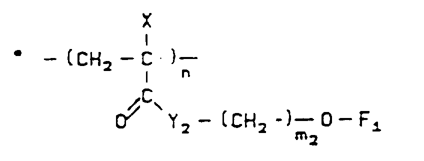 with
X = CH₃ or H
Y₂ = -O- or -NH-
2 ≤ m₂ ≤ 5
or

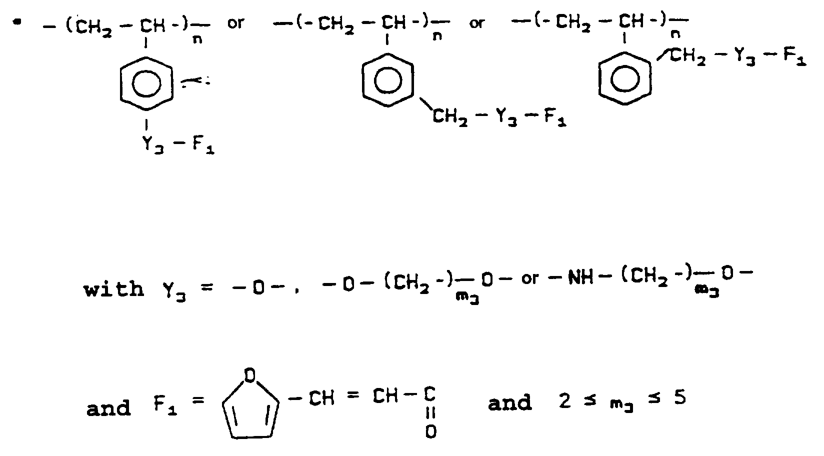

3. Material according to either of Claims 1 or 2, characterized in that the small molecule is transparent in the range 0.8 µm - 2 µm and corresponds to one of the following chemical formulae:

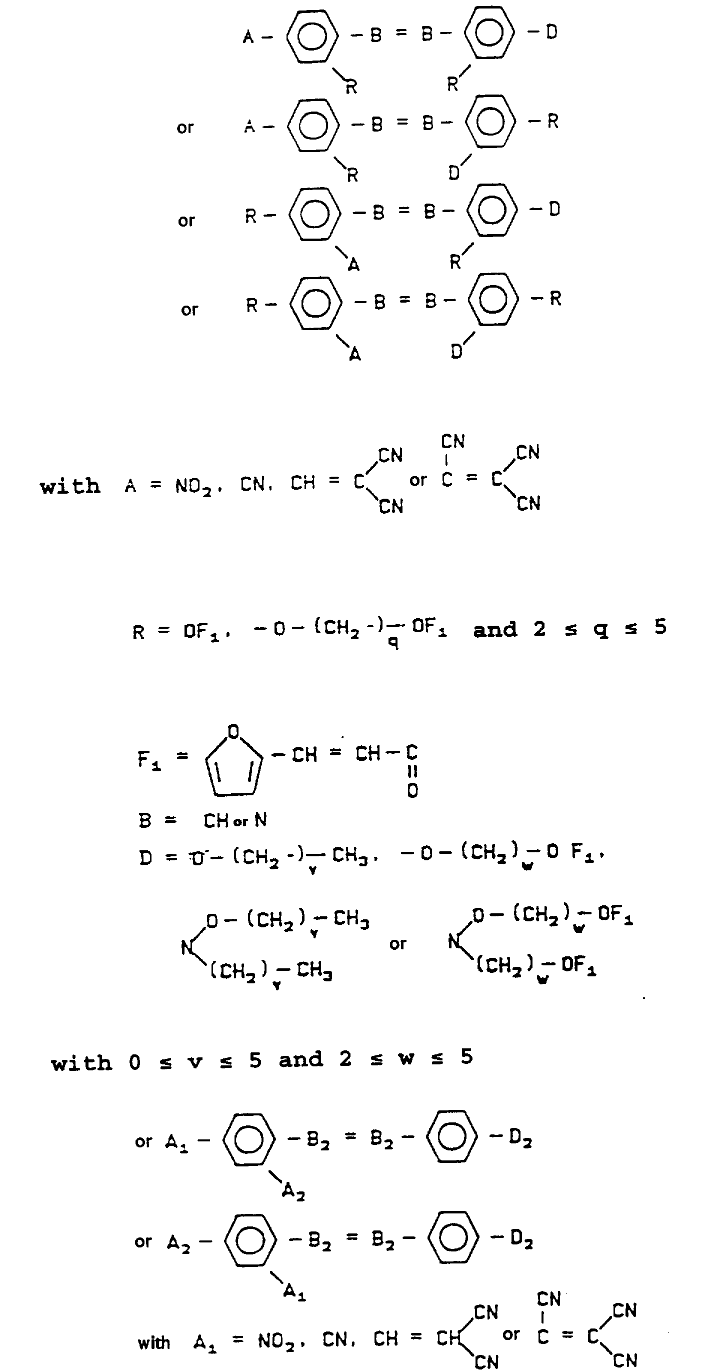

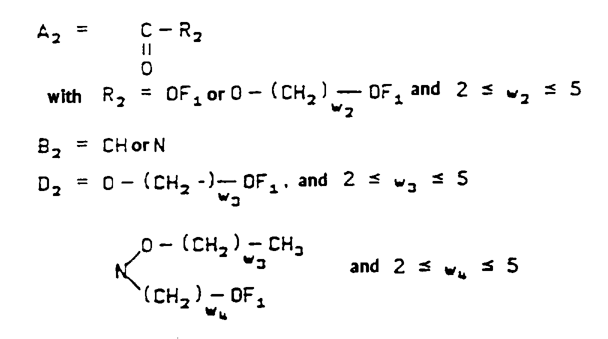

4. Material according to either of Claims 1 or 2, characterized in that the small molecule is transparent at wavelengths of greater than 300 nm and in that it corresponds to one of the following chemical formulae:

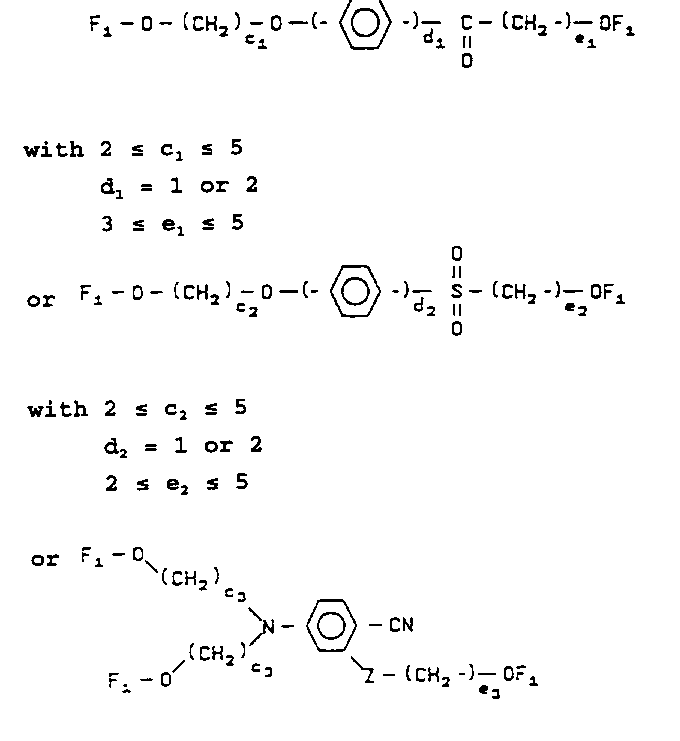

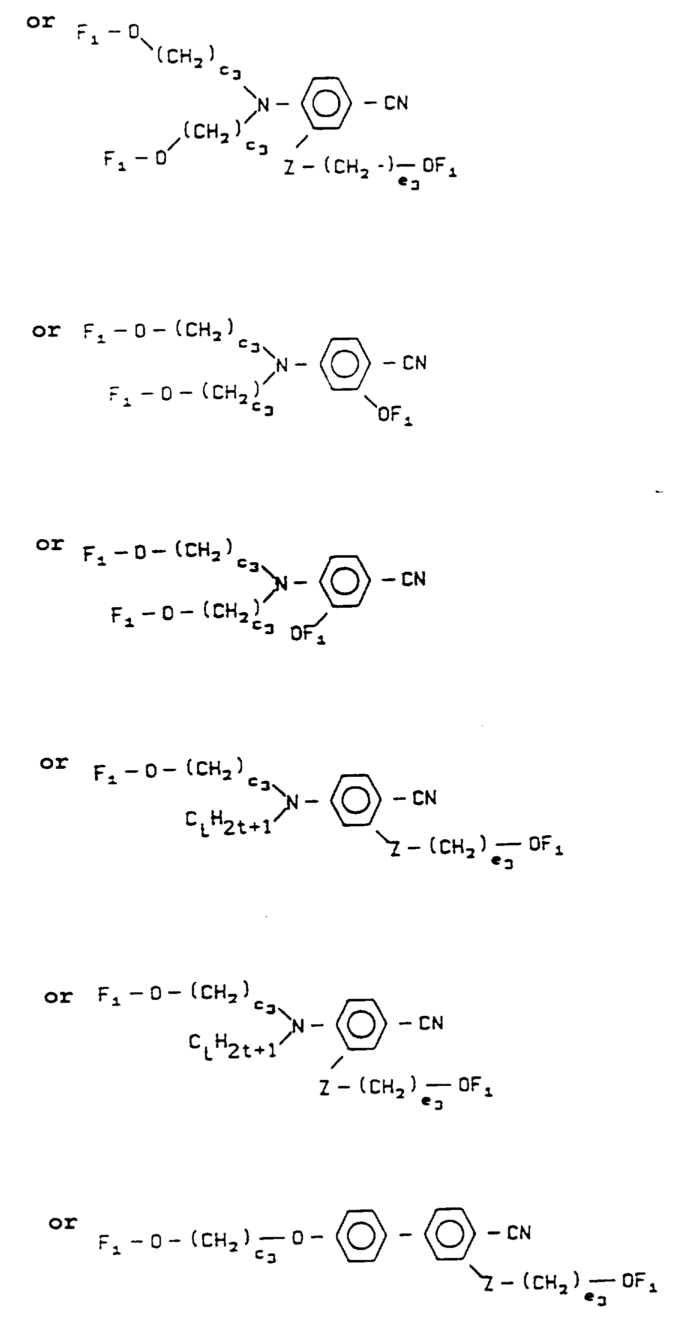

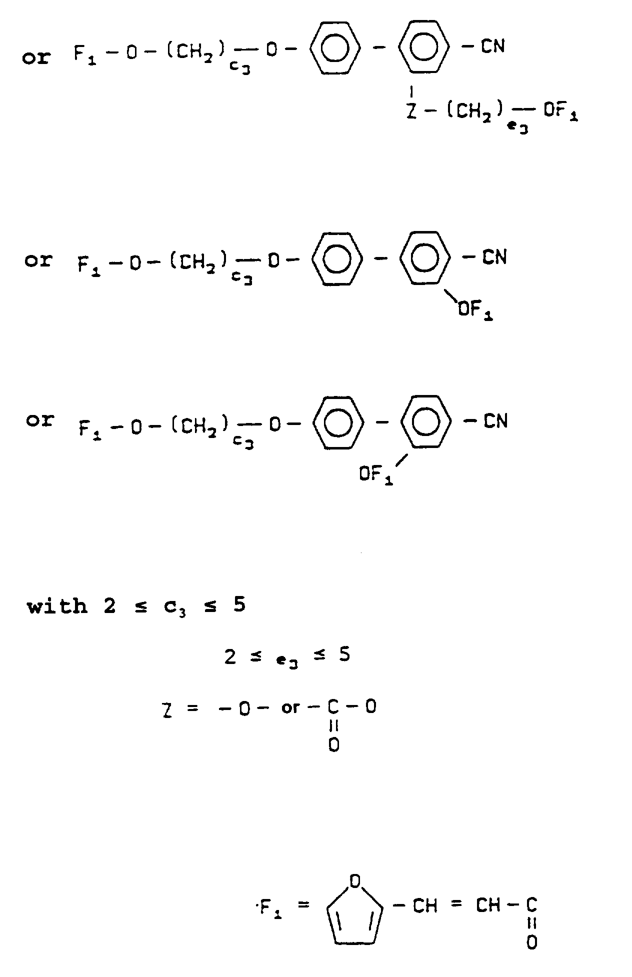

5. Material according to either of Claims 1 or 2, characterized in that the small molecule is transparent at wavelengths of greater than 350 nm and in that it corresponds to one of the following chemical formulae:

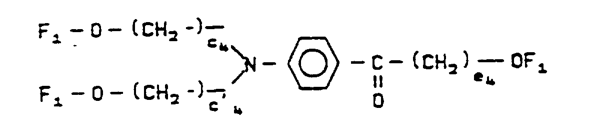

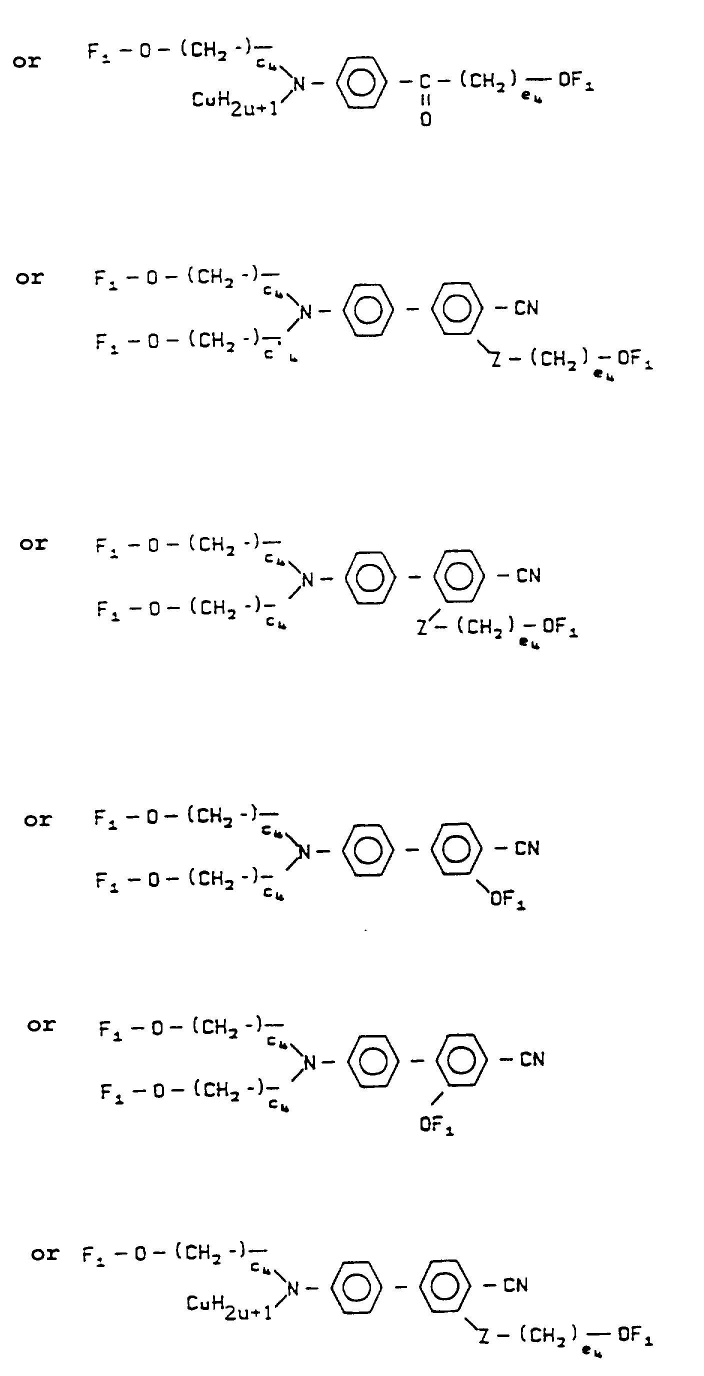

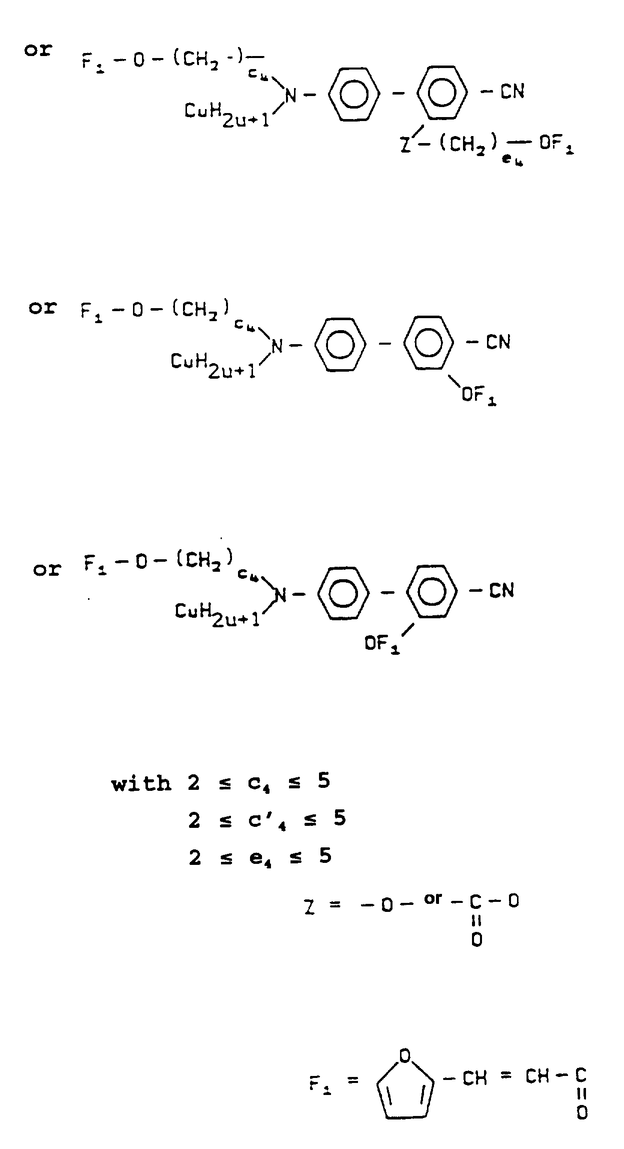

6. Process for the synthesis of a composite material which can be used in non-linear optics according to Claim 1 comprising the synthesis of poly(methacryloyloxypropyl furylacrylate), characterized in that it contains the following stages:
1. Synthesis of 3-bromopropyl methacrylate from the esterification of methacryloyl chloride in the presence of 3-bromopropanol;
2. Radical homopolymerization of 3-bromopropyl methacrylate in the presence of dimethylformamide (DMF) and of azobisisobutyronitrile (AIBN);
3. Esterification of the homopolymer obtained in Stage 2 in the presence of hexamethylphosphoramide (HMPA), of 1,8-diazabicyclo[5.4.0]undec-7-ene (DBU) and of furylacrylic acid.

7. Process for the synthesis of a composite material which can be used in non-linear optics according to Claim 1 comprising the synthesis of 2,2'-bis(furylacryloxy)-4-diethylamino-4'-nitroazobenzene, characterized in that it comprises the following stages:
1. Synthesis of furylacryloyl chloride in the presence of furylacrylic acid, of oxalyl chloride and of DMF;
2. Esterification of furylacryloyl chloride in the presence of tetrahydrofuran (THF) and of 2,2'-bis-(hydroxy)-4-diethylamino-4'-nitroazobenzene.
